(19) Europäisches Patentamt
European Patent Office
Office européen des brevets

(11) Veröffentlichungsnummer: **0 090 776**
**A1**

(12) # EUROPÄISCHE PATENTANMELDUNG

(21) Anmeldenummer: **83810124.4**

(22) Anmeldetag: **25.03.83**

(51) Int. Cl.³: **C 07 D 307/86**, A 01 N 47/24, C 07 C 161/00

(30) Priorität: **31.03.82 CH 1981/82**

(43) Veröffentlichungstag der Anmeldung: **05.10.83 Patentblatt 83/40**

(84) Benannte Vertragsstaaten: **BE CH DE FR GB LI NL**

(71) Anmelder: **CIBA-GEIGY AG, Patentabteilung Postfach, CH-4002 Basel (CH)**

(72) Erfinder: **Drabek, Jozef, Dr., Benkenstrasse 12, CH-4104 Oberwil (CH)**

(54) **Acylamidosulfenylcarbamate zur Bekämpfung von Insekten.**

(57) Mittel zur Bekämpfung von Insekten, insbesondere zur Bekämpfung von Pflanzen und Tiere befallenden Insekten, welches als aktive Komponente eine Verbindung der Formel I

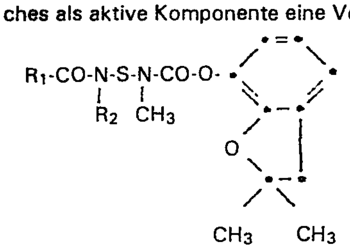

(I),

worin $R_1$ und $R_2$ geradkettiges oder verzweigtes $C_1$-$C_4$-Alkyl oder $C_3$-$C_4$-Cycloalkyl bedeuten, enthält, sowie Verfahren und neue Ausgangsprodukte zur Herstellung der Verbindungen der Formel I.

Neue Verbindungen der Formel Ia

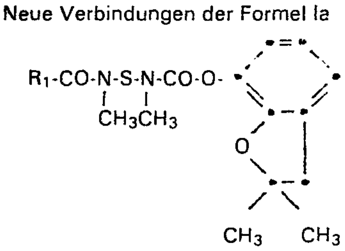

(Ia)

worin $R_1$ geradkettiges oder verzweigtes $C_2$-$C_4$-Alkyl oder Cyclopropyl bedeutet.

Verbindung der Formel II

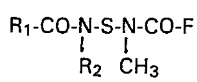

(II)

worin $R_1$ und $R_2$ geradkettiges oder verzweigtes $C_1$-$C_4$-Alkyl oder $C_3$-$C_4$-Cycloalkyl bedeuten.

CIBA-GEIGY AG
Basel (Schweiz)

5-13871/1+2

## Acylamidosulfenylcarbamate zur Bekämpfung von Insekten

Die vorliegende Erfindung betrifft insektizide Mittel enthaltend als insektiziden Wirkstoff Acylamidosulfenylcarbamate der nachstehenden Formel I sowie die Verwendung dieser Carbamte zur Bekämpfung von Insekten.

Die Erfindung betrifft weiterhin neue Wirkstoffe, d.h. Acylamidosulfenylcarbamate, die noch nicht in der Literatur vorbeschrieben sind.

Erfindungsgemäss wird ein insektizides Mittel vorgeschlagen, welches als aktive Komponente eine Verbindung der Formel I

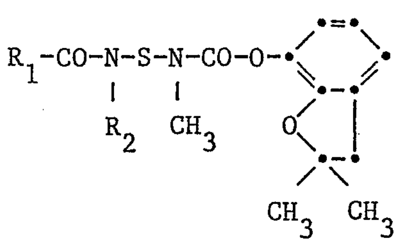

(I),

worin $R_1$ und $R_2$ geradkettiges oder verzweigtes $C_1$-$C_4$-Alkyl oder $C_3$-$C_4$-Cycloalkyl bedeuten, zusammen mit geeigneten Trägern und/oder anderen Zuschlagstoffen enthält.

Wegen ihrer Wirkung werden erfindungsgemässe Mittel bevorzugt, die Verbindungen der Formel I enthalten, worin $R_1$ geradkettiges oder verzweigtes $C_2$-$C_4$-Alkyl oder $C_3$-$C_4$-Cycloalkyl bedeutet, insbesondere solche, worin $R_2$ Methyl oder Aethyl, vorzugsweise Methyl, bedeutet.

Hervorzuheben sind auch diejenigen Mittel, die eine Verbindung der Formel I enthalten, in der $R_1$ Aethyl, n-Propyl, Isopropyl oder n-Butyl bedeutet.

Es ist bereits aus der US-Patentschrift No. 3.812.174 bekannt, dass Acyl-N-alkylamidosulfenyl-N'-alkyl-arylcarbamate insektizide Eigenschaften besitzen. Weiterhin werden in der US Patentschrift No. 4.006.231 N-Dialkylamidosulfenyl-N'-methyl-2,2-dimethyl-2,3-dihydrobenzofuranyl (7)]-carbamate als Pestizide, insbesondere als Insektizide, beschrieben. Alkoxycarbonyl-N-alkylamidosulfenyl-N'-methyl-[2,2-dimethyl-2,3-dihydrobenzofuranyl (7)]-carbamate mit insektizider Wirkung sind Gegenstand der deutschen Offenlegungsschrift No. 2812622. Die vorstehend als Wirkstoffe für die erfindungsgemässen Mittel angegebenen Verbindungen der Formel I sind in allgemeiner Form in der US Defensive Publication No. T 977 008 erwähnt, jedoch wird in dieser Veröffentlichung lediglich auf die nematiziden Eigenschaften dieser Verbindungen hingewiesen.

Demgegenüber wurde nun überraschenderweise gefunden, dass die als Wirkstoffe in den erfindungsgemässen Mitteln vorgeschlagenen Acyl-N-methylamidosulfenyl-N'-methyl-[2,2-dimethyl-2,3-dihydrobenzofuranyl (7)]-carbamate der vorstehenden Formel I gute insektizide Wirkung zeigen.

Besonders ausgeprägte insektizide Wirkung zeigen die Verbindungen der Formel Ia

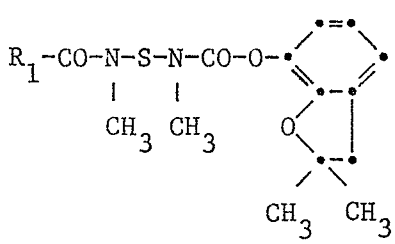

(Ia)

worin $R_1$ verzweigtes oder geradkettiges $C_2$-$C_4$-Alkyl oder Cyclopropyl bedeutet. Bevorzugt werden solche Verbindungen der Formel Ia, worin $R_1$ geradkettiges $C_2$-$C_4$-Alkyl bedeutet sowie solche, worin $R_1$ Aethyl, n-Propyl oder Isopropyl bedeutet.

Die Verbindungen der Formel (Ia) sind neu und werden als solche nicht in der vorerwähnten US Defensive Publication No. T 977 008 offenbart. Diese neuen Verbindungen bilden ebenfalls einen Gegenstand der vorliegenden Erfindung.

Die Verbindungen der Formel I bzw. Ia sind erfindungsgemäss nach dem folgenden neuartigen Verfahren erhältlich:

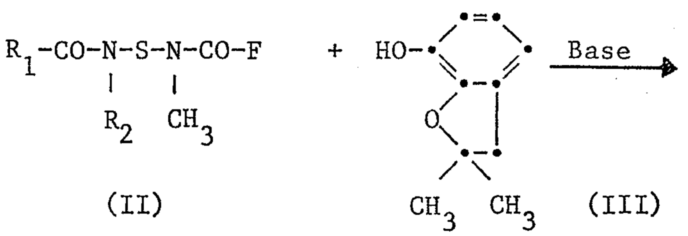


(II) (III)

I bzw. Ia

Die Verbindungen der Formel I bzw. Ia können weiterhin nach an sich bekannten Methoden, z.B. nach der folgenden Arbeitsweise, hergestellt werden:

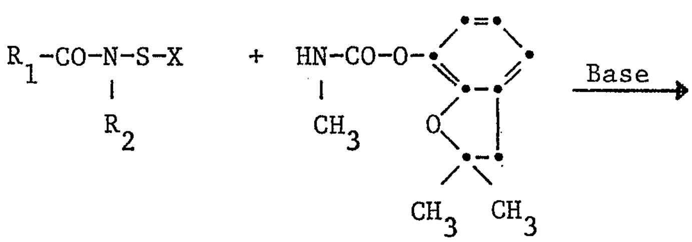


I bzw. Ia

(IV) (V)

In den Formeln II und IV haben $R_1$ und $R_2$ die für die Formel I bzw. Ia angegebenen Bedeutungen und X steht für ein Halogenatom, insbesondere für ein Chloratom.

Die vorstehenden Verfahren werden im allgemeinen bei einer Reaktionstemperatur zwischen -50°C und +130°C, vorzugsweise zwischen -10°C und +100°C, bei normalem oder leicht erhöhtem Druck und bevorzugt in Gegenwart eines gegenüber den Reaktionsteilnehmern inerten Lösungs- oder Verdünnungsmittels durchgeführt.

Als geeignete Basen für diese Verfahren kommen insbesondere tertiäre Amine, wie Trialkylamine, Pyridine und Dialkylaniline ferner Hydroxide, Oxide, Carbonate und Bicarbonate von Alkali- und Erdalkalimetallen sowie Alkalimetallalkoholate wie z.B. Kalium-tert.-butylat und Natrium-methylat in Betracht.

Als Lösungs- oder Verdünnungsmittel eignen sich z.B.Aether und ätherartige Verbindungen wie Diäthyläther, Di-isopropyläther, Dioxan, Tetrahydrofuran; aliphatische und aromatische Kohlenwasserstoffe, insbesondere Benzol, Toluol, Xylole; und Ketone wie Aceton, Methyläthylketon und Cyclohexanon.

Die Ausgangsstoffe der Formeln III bis V sind bekannt oder können, falls sie neu sind, analog bekannten Arbeitsweisen hergestellt werden. (vgl. z.B. US-Patentschrift Nr. 3.320.286, USDefensive-Publication Nr. T 977008, britische Patentschrift Nr. 999.128). Die Ausgangsstoffe der Formel II sind neuartige Verbindungen, die ebenfalls einen Gegenstand der vorliegenden Erfindung bilden.

Die Verbindungen der Formel I bzw. Ia und die sie enthaltenden Mittel, eignen sich u.a. zur Bekämpfung von Insekten der Ordnungen: Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera.

Es ist hervorzuheben, dass die erfindungsgemäss genannten Verbindungen sich sowohl durch eine stark ausgeprägte systemische als auch Kontakt-Wirkung gegen saugende Insekten, insbesondere gegen saugende Insekten der Ordnung Homoptera und vor allem gegen Insekten der Familie Aphididae (wie z.B. Aphis fabae, Aphis craccivora und Myzus persicae), welche sich mit bekannten Mitteln nur schwierig bekämpfen lassen, auszeichnen.

Neben ihrer Wirkung gegenüber Mückenlarven können Verbindungen der Formel I bzw. Ia auch zur Bekämpfung von pflanzenschädigenden Frassinsekten in Zier- und Nutzpflanzungen, insbesondere in Baumwollkulturen (z.B. gegen Anthomus grandis, Spodoptera littoralis und Heliothis virescens), sowie in Obst- und Gemüsekulturen (z.B. gegen Laspeyresia pomonella, Leptinotarsa decemlineata und Epilachna varivestis) eingesetzt werden. Die Verbindungen der Formel I bzw. Ia zeichnen sich durch eine gute Wirkung gegen larvale Insektenstadien und Nymphen insbesondere von fressenden Schadinsekten, aus; zu erwähnen ist ferner ihre breite ovizide und ovolarvizide Wirkung. Werden Verbindungen der Formel I bzw. Ia von adulten Insekten-Stadien mit dem Futter aufgenommen, so ist in vielen Fällen, insbesondere bei Coleopteren, wie z.B. Anthonomus grandis, eine verminderte Ei-Ablage und/oder reduzierte Schlupfrate festzustellen. Weiterhin können die Verbindungen der Formel I bzw. Ia erfolgreich gegen Chilo suppressalis und gegen pflanzenschädigende Zikaden, wie Laodelphax striatellus sowie Nilaparvata lugens, speziell in Reis-Kulturen, eingesetzt werden.

Die Verbindungen der Formel I bzw. Ia können ferner zur Bekämpfung von Ektoparasiten, wie Lucilia sericata, an Haus- und Nutztieren eingesetzt werden, z.B. durch Tier-, Stall- und Weidebehandlung.

Die Wirkung der Verbindungen der Formel I bzw. Ia sowie der sie enthaltenden Mittel lässt sich durch Zusatz von anderen Insektiziden und/oder Akariziden wesentlich verbreitern und an gegebene Umstände anpassen. Als Zusätze kommen z.B. folgende Wirkstoffe in Betracht: organische Phosphorverbindungen, Nitrophenole und Derivate, Formamidine, Harnstoffe, Carbamate, chlorierte Kohlenwasserstoffe und Bacillus thuringiensis-Präparate.

Mit besonderem Vorteil kann man die Verbindungen der Formel I bzw. Ia auch mit Substanzen kombinieren, welche einen pestizid verstärkenden Effekt ausüben. Beispiele solcher Verbindungen sind u.a.: Piperonylbutoxid, Propinyläther, Propinyloxime, Propinylcarbamate und Propinylphosphonate, 2-(3,4-Methylendioxyphenoxy)-3,6,9-trioxa-undecan oder S,S,S-Tributylphosphorotrithioate.

Die gute insektizide Wirkung der erfindungsgemäss genannten Verbindungen der Formel I bzw. Ia entspricht einer Abtötungsrate (Mortalität) von mindestens 50-60% der erwähnten Schadinsekten.

Die Verbindungen der Formel I bzw. Ia werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierungen, d.h. die den Wirkstoff der Formel I bzw. Ia oder Kombinationen dieser Wirkstoffe mit andern Insektiziden oder Akariziden, und gegebenenfalls einen festen oder flüssigen Zusatzstoff enthaltenden Mittel , Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther,

Ketone, wie Cyclohexanon, stark polare Lösungsmittel, wie N-Methyl-2-pyrrolidon, Dimethylsulfoxid oder Dimethylformamid, sowie gegebenenfalls epoxydierte Pflanzenöle, wie epoxydiertes Kokosnussöl oder Sojaöl oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Stoffe, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur, wie insbesondere Dolomit oder zerkleinerte Pflanzenrückstände verwendet werden.

Als oberflächenaktive Verbindungen kommen je nach der Art des zu formulierenden Wirkstoffs der Formel I bzw. Ia oder der Kombination dieser Wirkstoffe mit anderen Insektiziden oder Akariziden nichtionogene, kation- und/oder antionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze oder Oel- oder Stearinsäure, oder von natürlichen Fettsäuregemischen, die z.B. aus Kokosnuss- oder Talgöl gewonnen werden können. Ferner sind auch die Fettsäure-methyl-taurinsalze sowie modifizierte und nicht-modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxyd-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxyd-Adduktes in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiter geeignete nichtionische Tenside sind die wasserlöslichen 20 bis 250 Aethylenglykoläthergruppen und 10 bis 100 Propylenglykoläthergruppen enthaltend Polyäthylenoxidaddukte an Polypropylenglykol, Aethylendiaminopolypropylenglykol und Alkylpolypropylenglykol mit 1 bis 10 Kohlenstoffatomen in der Alkylkette. Die genanten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxydaddukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylenammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ringwood, New Jersey, 1979.

Die pestiziden Zubereitungen enthalten in der Regel 0,1 bis 99%, insbesondere 0,1 bis 95%, Wirkstoff der Formel I bzw. Ia oder Kombinationen dieser Wirkstoffe mit andern Insektiziden oder Akariziden, 1 bis 99,9% eines festen oder flüssigen Zusatzstoffes und 0 bis 25%, insbesondere, 0,1 bis 20%, eines Tensides. Während als Handelsware eher konzentrierte Mittel bevorzugt werden, verwendet der Endverbraucher in der Regel Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Formulierungsbeispiele für flüssige Wirkstoffe der Formel I oder Kombinationen dieser Wirkstoffe mit andern Insektiziden oder Akariziden (% = Gewichtsprozent)

| 1. Emulsions-Konzentrate | a) | b) | c) |
|---|---|---|---|
| Wirkstoff resp. Wirkstoffkombination | 25% | 40% | 50% |
| Ca-Dodecylbenzolsulfonat | 5% | 8% | 6% |
| Ricinusölpolyäthylenglykoläther (36 Mol AeO) | 5% | - | - |
| Tributylphenolpolyäthylenglykoläther (30 Mol AeO) | - | 12% | 4% |
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentration hergestellt werden.

| 2. Lösungen | a) | b) | c) | d) |
|---|---|---|---|---|
| Wirkstoff resp. Wirkstoffkombination | 80% | 10% | 5% | 95% |
| Aethylenglykolmonomethyläther | 20% | - | - | - |
| Polyäthylenglykol MG 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190°C) | - | - | 94% | - |

Die Lösungen sind zur Anwendung in Form kleinster Tropfen geeignet.

3. Granulate

| | a) | b) |
|---|---|---|
| Wirkstoff resp. Wirkstoffkombination | 5% | 10% |
| Kaolin | 94% | - |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

4. Stäubemittel

| | a) | b) |
|---|---|---|
| Wirkstoff resp. Wirkstoffkombination | 2% | 5% |
| Hochdisperse Kieselsäure | 1% | 5% |
| Talkum | 97% | - |
| Kaolin | - | 90% |

Durch inniges Vermischen der Trägerstoffe mit dem Wirkstoff erhält man gebrauchsfertige Stäubemittel.

Formulierungsbeispiele für feste Wirkstoffe der Formel I resp. Kombinationen dieser Wirkstoffe mit andern Insektiziden oder Akariziden (% = Gewichtsprozent)

5. Spritzpulver

| | a) | b) | c) |
|---|---|---|---|
| Wirkstoff oder Wirkstoffkombination | 25% | 50% | 45% |
| Na-Ligninsulfonat | 5% | 5% | - |
| Na-Laurylsulfat | 3% | - | 5% |
| Na-Diisobutylnaphthalinsulfonat | - | 6% | 10% |
| Octylphenolpolyäthylenglykoläther (7-8 Mol AeO) | - | 2% | - |
| Hochdisperse Kieselsäure | 5% | 10% | 10% |
| Kaolin | 62% | 27% | - |

Der Wirkstoff oder die Wirkstoffkombination werden mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

6. Emulsions-Konzentrat

| | |
|---|---|
| Wirkstoff oder Wirkstoffkombination | 10% |
| Octylphenolpolyäthylenglykoläther (4-5 Mol AeO) | 3% |
| Ca-Dodecylbenzolsulfonat | 3% |
| Ricinusölpolyglykoläther (36 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| 7. Stäubemittel | a) | b) |
|---|---|---|
| Wirkstoff oder Wirkstoffkombination | 5% | 8% |
| Talkum | 95% | - |
| Kaolin | - | 92% |

Man erhält anwendungsfertige Stäubemittel, indem der Wirkstoff mit dem Träger vermischt und auf einer geeigneten Mühle vermahlen wird.

8. Extruder-Granulat

| | |
|---|---|
| Wirkstoff oder Wirkstoffkombination | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert, granuliert und anschliessend im Luftstrom getrocknet.

9. Umhüllungs-Granulat

| | |
|---|---|
| Wirkstoff oder Wirkstoffkombination | 3% |
| Polyäthylenglykol (MG 200) | 3% |
| Kaolin | 94% |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

10. Suspensions-Konzentrat

| | |
|---|---|
| Wirkstoff oder Wirkstoffkombination | 40% |
| Aethylenglykol | 10% |
| Nonylphenolpolyäthylenglykoläther (15 Mol AeO) | 6% |
| Na-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 1:

Herstellung der Ausgangsverbindungen:

In einer Polyäthylenapparatur werden 200 ml Toluol vorgelegt und auf -50°C abgekühlt. Bei dieser Temperatur werden im Toluol unter Rühren 13,2 g Fluorwasserstoffsäure gelöst und zu dieser Lösung bei -50 bis -30°C 39,1 ml Methylisocyanat während 15 Minuten zugetropft. Das Reaktionsgemisch wird während zwei Stunden weiter gerührt und dann mit 800 ml Toluol verdünnt. Zu dieser Lösung werden während 10 Minuten 100,6 g N-Chlorsulfenylpropionsäuremethylamid und anschliessend während 30 Minuten 90,9 ml Triäthylamin bei -10°C zugetropft. Das Reaktionsgemisch wird anschliessend während 24 Stunden bei Raumtemperatur und dann 8 Stunden lang bei 50-60°C gerührt.

Nach dem Abkühlen wird das Gemisch abgesaugt. Das erhaltene Filtrat wird eingeengt und der sich ergebende Rückstand im Hochvakuum destilliert. Es wird die Verbindung der Formel

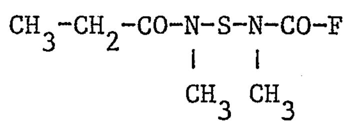

vom Sdp. 78-83°C/0,07 - 0,1 Torr erhalten.

In entsprechender Weise werden die folgenden Verbindungen der Formel II hergestellt:

| $R_1$ | $R_2$ | physikalische Daten |
|---|---|---|
| $CH_3-$ | $CH_3-$ | Sdp. 64-70°C / 0,05 Torr |
| $CH_3-(CH_2)_2-$ | $CH_3-$ | Sdp. 96-98°C / 0,08 Torr |
| $(CH_3)_2CH-$ | $CH_3-$ | Sdp. 82-87°C / 0,08 Torr |
| $CH_3-(CH_2)_3-$ | $CH_3-$ | |
| $CH_3-CH_2-CH(CH_3)-$ | $CH_3-$ | |
| $(CH_3)_3C-$ | $CH_3-$ | |
| $(CH_3)_2CH-CH_2-$ | $CH_3-$ | |
| 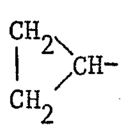 | $CH_3-$ | |

Herstellung von Isobutyryl-N-methylamidosulfenyl-N'-methyl-[2,2-dimethyl-2,3-dihydrobenzofuranyl(7)]-carbamat:

Zu einer Lösung von 8,67 ml 2,2-Dimethyl-2,3-dihydro-7-hydroxybenzofuran 11,05 ml Triäthylamin und 0,2 g Dimethylaminopyridin in 100 ml Toluol werden bei einer Temperatur von 0 bis 10°C 11,0 g Isobutyryl-N-methylamidosulfenyl-N'-methylcarbamoylfluorid in 10 ml Toluol während 20 Minuten zugetropft. Das Reaktionsgemisch wird während 5 Stunden bei Raumtemperatur und sodann 12 Stunden bei 70 bis 80°C gerührt. Nach Abdestillieren der organischen Lösungsmittel wird das Reaktionsgemisch in Toluol aufgenommen und im Scheidetrichter nacheinander mit 100 ml Wasser, 100 ml 1N Salzsäure, 100 ml 1N Natriumbicarbonatlösung und 100 ml 20%-iger Kochsalzlösung gewaschen. Die abgetrennte organische Phase wird über Natriumsulfat getrocknet und das Lösungsmittel abdestilliert. Als Destillationsrückstand wird die Titelverbindung als Oel, $n_D^{40}$ = 1,5260, der Formel

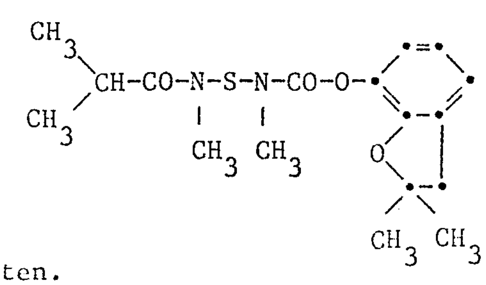

(Verbindung Nr. 1)

erhalten.

Analog den vorstehend beschriebenen Arbeitsweisen werden die folgenden Verbindungen der Formel I bzw. Ia erhalten:

| Verbindung Nr. | $R_1$ | $R_2$ | physikalische Daten |
|---|---|---|---|
| 2 | $CH_3-(CH_2)_2-$ | $CH_3-$ | $n_D^{40} = 1,5278$ |
| 3 | $CH_3-CH_2-$ | $CH_3-$ | $n_D^{45} = 1,5380$ |
| 4 | $CH_3-(CH_2)_3-$ | $CH_3-$ | $n_D^{30} = 1,5280$ |
| 5 | $CH_3-CH_2-CH(CH_3)-$ | $CH_3-$ | |
| 6 | $(CH_3)_3C-$ | $CH_3-$ | |
| 7 | $(CH_3)_2CH-CH_2-$ | $CH_3-$ | |
| 8 | 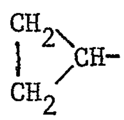 | $CH_3$ | |

Beispiel 2: Wirkung gegen Aëdes aegypti: Auf die Oberfläche von 150 ml Wasser, das sich in einem Behälter befindet, wird so viel einer 0,1%igen acetonischen Lösung des Wirkstoffes pipettiert, dass Konzentrationen von je 800 und 400 ppm erhalten werden. Nach Verdunsten des Acetons wird der Behälter mit 30-40 2-tägigen Aëdes-Larven beschickt. Nach 1, 2 und 5 Tagen wird die Mortalität geprüft.

Verbindungen der Formel I bzw. Ia gemäss Beispiel 1 zeigt in diesem Test gute Wirksamkeit gegen Aëdes aegypti.

Beispiel 3: Insektizide Kontakt-Wirkung gegen Aphis craccivora:

In Wasser angezogene Erbsenkeimlinge werden vor dem Versuchsbeginn je mit ca. 200 Individuen der Spezies Aphis craccivora besiedelt. Die so behandelten Pflanzen werden 3 Tage später mit Lösungen enthaltend 3,0 und 0,75 ppm der zu prüfenden Verbindung aus 30 cm Distanz bis zur Tropfnässe besprüht. Man verwendet pro Test-Verbindung und pro Konzentration zwei Pflanzen; Auswertung der erzielten Abtötungsrate erfolgt nach weiteren 24 Stunden.

Beispiel 4: Insektizide systemische Wirkung gegen Myzus persicae:

Bewurzelte Rettichpflanzen werden in Töpfe, welche 600 ccm Erde enthalten, verpflanzt und anschliessend mit einer Population von Myzus persicae infiziert. Nach drei Tagen werden die infizierten Pflanzen mit je 50 ml einer wässrigen Zubereitung der zu prüfenden Verbindung (erhalten aus einem 25%-igen emulgierbaren Spritzpulver) in einer Konzentration von 3,0 und 12,5 ppm direkt auf die Erde angegossen.

Der Versuch wird im Gewächshaus bei 28°C durchgeführt. Pro Konzentrationsdosis und Testsubstanz werden zwei Pflanzen, je eine in einem separaten Topf, verwendet. Die Auswertung der erzielten Abtötung erfolgt 48 Stunden nach Angiessen der Pflanzen.

Beispiel 5: Insektizide Frassgift-Wirkung gegen Laspeyresia pomonella:

Aepfel der Sorte Golden Delicious werden mit wässrigen Emulsionszubereitungen, enthaltend 3,0, 12,5, 50, 100 und 200 ppm der zu prüfenden Verbindung, besprüht.

Nach dem Antrocknen des Spritzbelages werden die Früchte mit Larven der Spezies Laspeyresia pomonella ($L_1$-Stadium) besetzt, und zwar pro Apfel zwei Testtiere. Man verwendet pro Versuchsverbindung zwei Äpfel. Der Versuch wird bei 25°C und 60% relativer Luftfeuchtigkeit durchgeführt. Die Auswertung der erzielten Abtötungsrate erfolgt nach 4 Tagen.

Beispiel 6: Wirkung auf Laspeyresia pomonella (Eier):

Abgelegte Eier von Laspeyresia pomonella, die nicht älter als 24 Stunden sind, werden auf Filterpapier für 1 Minute in eine acetonischwässrige Lösung enthaltend 12,5, 200 oder 400 ppm des zu prüfenden Wirkstoffes eingetaucht. Nach dem Antrocknen der Lösung werden die Eier in Petrischalen ausgelegt und bei einer Temperatur von 28°C belassen. Nach 6 Tagen wird der prozentuale Schlupf aus den behandelten Eiern bewertet.

Beispiel 7: Wirkung auf Heliothis virescens (Eier):

Entsprechende Mengenanteile einer benetzbaren pulverförmigen Formulierung, enthaltend 25 Gew.-% des zu prüfenden Wirkstoffes, werden mit jeweils soviel Wasser vermischt, dass sich wässrige Emulsionen von ansteigender Wirkstoffkonzentration, d.h. von 12,5, 50, 100 und 400 ppm, ergeben.

In diese wirkstoffhaltigen Emulsionen werden eintägige Eigelege von Heliothis auf Cellophan während drei Minuten eingetaucht und dann auf Rundfiltern abgenutscht. Die so behandelten Gelege werden in Petrischalen ausgelegt und in der Dunkelheit aufbewahrt. Nach 6 bis 8 Tagen wird die Schlupfrate im Vergleich zu unbehandelten Kontrollen festgestellt. Zur Auswertung wird die zur 100%igen Abtötung der Eier erforderliche minimale Wirkstoffkonzentration bestimmt.

Beispiel 8: Wirkung gegen Anthonomus grandis:

Zwei eingetopfte Baumwollpflanzen im 6-Blattstadium werden mit einer wässrigen, benetzungsfähigen Emulsions-Zubereitung enthaltend 50, 100 und 400 ppm des zu prüfenden Wirkstoffes besprüht. Nach dem Antrocknen des Spritzbelages (etwa 1,5 Stunden) wird jede Pflanze mit 10 adulten Käfern (Anthonomus grandis) besiedelt. Plastikzylinder, deren obere Oeffnungen mit Gaze abgedeckt sind, werden dann über die behandelten, mit den Testtieren besiedelten Pflanzen gestülpt, um ein Abwandern der Käfer zu verhindern. Die behandelten Pflanzen werden bei 25°C und etwa 60% relativer Luftfeuchtigkeit gehalten. Die Auswertung erfolgt nach 2, 3, 4 und 5 Tagen unter Zugrundelegung der

prozentualen Mortalität der eingesetzten Testtiere (% Rückenlage) sowie der Antifeedant-Wirkung gegenüber unbehandelten Kontrollansätzen.

Beispiel 9: Reproduktions-Beeinflussung von Anthonomus grandis:

Adulte Anthonomus grandis, die nach dem Schlupf nicht älter als 24 Stunden sind, werden in Gruppen zu jeweils 25 Käfern in Käfige mit Gitterwänden überführt. Die mit den Käfern besetzten Käfige werden sodann während 5 bis 10 Sekunden in eine acetonische Lösung, enthaltend 1,0 Gew.-% des zu prüfenden Wirkstoffes, eingetaucht.

Nachdem die Käfer wieder trocken sind, werden sie zur Kopulation und Eiablage in abgedeckte und Futter enthaltende Schalen eingesetzt. Abgelegte Eier werden zwei- bis dreimal wöchentlich mit fliessendem Wasser ausgeschwemmt, gezählt, durch zwei bis dreistündiges Einlegen in ein wässriges Desinfektionsmittel desinfiziert und dann in Schalen, die eine geeignete Larvaldiät enthalten, deponiert. Nach 7 Tagen wird untersucht, ob sich aus den deponierten Eiern Larven entwickelt haben.

Zur Ermittlung der Dauer des die Reproduktion beeinflussenden Effektes der zu prüfenden Wirkstoffe wird die Eiablage der Käfer während eines Zeitraumes von etwa vier Wochen überprüft. Die Bonitierung erfolgt anhand der Verminderung der Anzahl abgelegter Eier und der daraus geschlüpften Larven im Vergleich zu unbehandelten Kontrollen.

Verbindungen der Formel I bzw. Ia gemäss Beispiel 1 zeigen eine gute reproduktionsreduzierende Wirkung im obigen Test.

Beispiel 10: Wirkung gegen Laodelphax striatellus und Nilaparvata lugens (Nymphen):

Der Test wird an wachsenden Pflanzen durchgeführt. Dazu werden jeweils 4 Reispflanzen (Dicke des Stengels 8 mm) mit einer Höhe von ca. 20 cm in Töpfe (Durchmesser von 8 cm) eingepflanzt.

Die Pflanzen werden auf einem Drehteller mit jeweils 100 ml einer acetonischen Lösung enthaltend 50, 200, 400 oder 800 ppm Wirkstoff besprüht. Nach dem Antrocknen des Spritzbelages erfolgt die Besiedlung jeder Pflanze mit je 20 Nymphen der Testtiere im dritten Stadium. Um die Zikaden am Entweichen zu hindern, wird über die besiedelten Pflanzen jeweils ein Glaszylinder gestülpt und dieser mit einem Gaze-Deckel abgedeckt. Die Nymphen werden bis zum Erreichen des folgenden Entwicklungsstadiums über 10 Tage an der behandelten Pflanze gehalten. Die Auswertung auf %-Mortalität erfolgt 1, 4 und 8 Tage nach der Behandlung.

Mit Verbindungen der Formel I bzw. Ia gemäss Beispiel 1 kann gegen Nymphen von Laodelphax striatellus und Nilaparvata lugens gute Wirksamkeit erzielt werden.

Beispiel 11: Wirkung gegen Laodelphax striatellus und Nilaparvata lugens (ovizid):

Der Test wird an wachsenden Pflanzen durchgeführt. Jeweils 4 Reispflanzen (Dicke des Stengels 8 mm, Höhe ca. 20 cm) werden in Töpfe (Durchmesser von 8 cm) eingepflanzt.

Die Pflanzen werden auf einer Drehtellervorrichtung mit jeweils 100 ml einer acetonischen Lösung enthaltend 400 oder 800 ppm Wirkstoff besprüht. Nach dem Antrocknen des Spritzbelages wird jede Pflanze mit 3 adulten Weibchen besiedelt. Um die Tiere am Entweichen zu hindern, wird über jede besiedelte Pflanze ein Glaszylinder gestülpt und dieser mit einem Gaze-Deckel abgedeckt. Die Weibchen bleiben 4 Tage zur Eiablage auf der behandelten Pflanze und werden dann entfernt.

Etwa 8 Tage nach Besiedlung sind die jungen Zikaden geschlüpft, und es erfolgt die Auszählung. Aus dem Vergleich der Anzahl geschlüpfter Larven auf den unbehandelten Pflanzen zu den auf unbehandelten Kontrollpflanzen geschlüpften Tieren wird die prozentuale Mortalität berechnet.

Verbindungen der Formel I bzw. Ia gemäss Beispiel 1 zeigen in obigem Test gute ovizide Wirkung.

Biologische Ergebnisse:

Die nachstehende Tabelle zeigt Ergebnisse insektizider Prüfungen erfindungsgemässer Verbindungen auf der Basis obiger biologischer Beispiele. Die Auswertung der Versuche erfolgt anhand der erhaltenen %-Mortalität unter Verwendung des folgenden Bewertungs-Index:

A: 80-100% Mortalität bei einer Konzentration von 0,75 ppm der geprüften Verbindung.

B: 80-100% Mortalität bei einer Konzentration von 3,0 ppm der geprüften Verbindung

C: 80-100% Mortalität bei einer Konzentration von 12,5 ppm der geprüften Verbindung.

D: 80-100% Mortalität bei einer Konzentration von 50 ppm der geprüften Verbindung

E: 80-100% Mortalität bei einer Konzentration von 100 ppm der geprüften Verbindung

F: 80-100% Mortalität bei einer Konzentration von 200 ppm der geprüften Verbindung

G: 80-100% Mortalität bei einer Konzentration von 400 ppm der geprüften Verbindung

| Verbindung Nr. | insektizide Wirksamkeit | | | | | | |
|---|---|---|---|---|---|---|---|
| | Aphis croccivora-Direktspray (Beispiel 3) | Myzus persicae-systemisch (Beispiel 4) | Laspeyresia pomonella-Larven (Beispiel 5) | Laspeyresia pomonella-Eier (Beispiel 6) | Heliothis virescens-Eier (Beispiel 7) | Anthonomus grandis-Adulte (Beispiel 8) | Laodelphax striatellus-Nymphen (Beispiel 10) |
| 1 | A | C | C | C | D | E | D |
| 2 | A | C | D | C | C | E | D |
| 3 | - | - | D | - | - | G | D |
| 4 | - | B | B | F | D | D | - |

Patentansprüche:

1. Mittel zur Bekämpfung von Insekten, welches als aktive Komponente eine Verbindung der Formel I

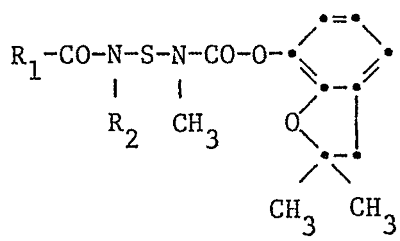

(I),

worin $R_1$ und $R_2$ geradkettiges oder verzweigtes $C_1$-$C_4$-Alkyl oder $C_3$-$C_4$-Cycloalkyl bedeuten, zusammen mit geeigneten Trägern und/oder anderen Zuschlagstoffen enthält.

2. Mittel nach Anspruch 1 enthaltend als aktive Komponente eine Verbindung der Formel I, worin $R_1$ geradkettiges oder verzweigtes $C_2$-$C_4$-Alkyl oder $C_3$-$C_4$-Cycloalkyl bedeutet.

3. Mittel nach Anspruch 1 oder 2 enthaltend als aktive Komponente eine Verbindung der Formel I, worin $R_2$ Methyl oder Aethyl bedeutet.

4. Mittel nach Anspruch 1 oder 2 enthaltend als aktive Komponente eine Verbindung der Formel I, worin $R_2$ Methyl bedeutet.

5. Mittel nach Anspruch 2 bis 4, enthaltend als aktive Komponente eine Verbindung der Formel I, worin $R_1$ Aethyl, n-Propyl, Isopropyl oder n-Butyl bedeutet.

6. Mittel nach Anspruch 1 enthaltend als aktive Komponente die Verbindung der Formel

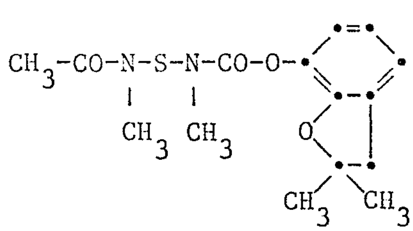

.

7. Mittel nach Anspruch 2 enthaltend als aktive Komponente die Verbindung der Formel

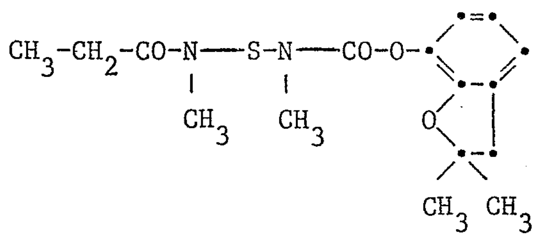

.

8. Mittel nach Anspruch 2 enthaltend als aktive Komponente die Verbindung der Formel

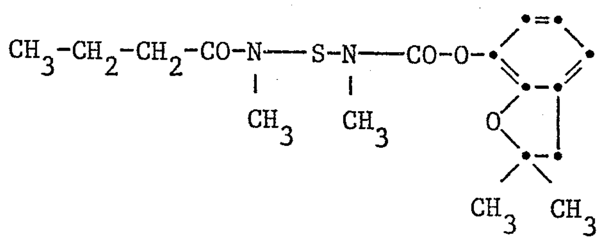

.

9. Mittel nach Anspruch 2 enthaltend als aktive Komponente die Verbindung der Formel

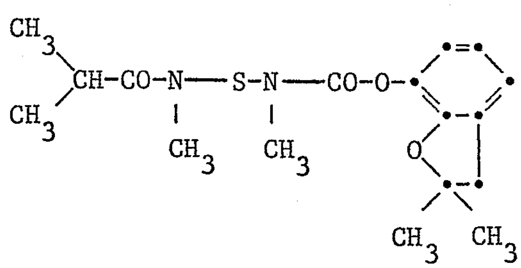

.

10. Mittel nach Anspruch 2 enthaltend als aktive Komponente die Verbindung der Formel

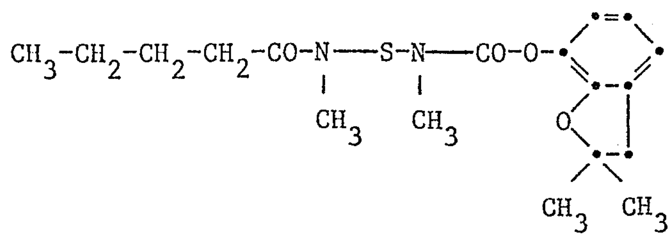

.

11. Verwendung einer Verbindung der Formel I

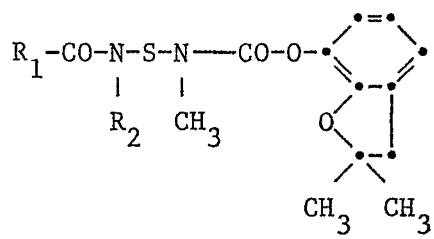

(I),

worin $R_1$ und $R_2$ geradkettiges oder verzweigtes $C_1$-$C_4$-Alkyl oder $C_3$-$C_4$-Cycloalkyl bedeuten, zur Bekämpfung von Insekten.

12. Verwendung nach Anspruch 11 zur Bekämpfung pflanzenschädigender Insekten.

13. Verwendung nach Anspruch 12 zur Bekämpfung von Eiern und/oder larvaler Stadien pflanzenschädigender Insekten.

14. Verbindung der Formel Ia

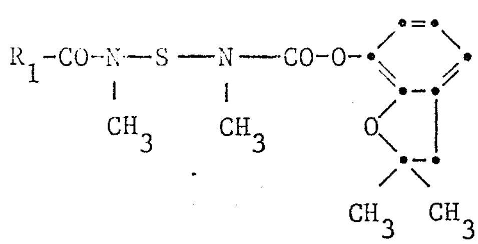

(Ia)

worin $R_1$ geradkettiges oder verzweigtes $C_2$-$C_4$-Alkyl oder Cyclopropyl bedeutet.

15. Verbindung der Formel Ia nach Anspruch 14, worin $R_1$ geradkettiges $C_2$-$C_4$-Alkyl bedeutet.

16. Verbindung der Formel Ia nach Anspruch 14, worin $R_1$ Aethyl, n-Propyl oder Isopropyl bedeutet.

17. Verbindung nach Anspruch 16 der Formel

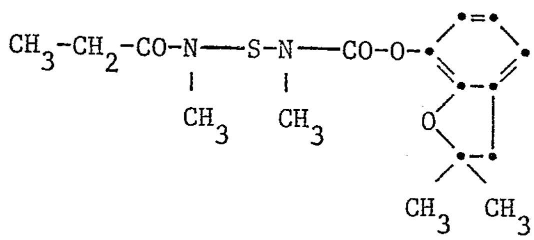

18. Verbindung nach Anspruch 16 der Formel

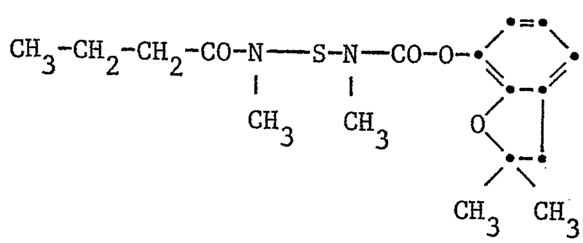

19. Verbindung nach Anspruch 16 der Formel

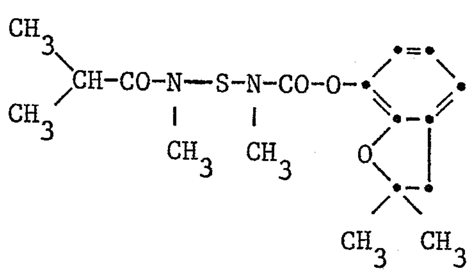

20. Verbindung nach Anspruch 15 der Formel

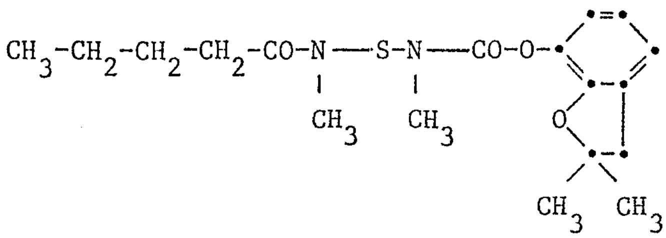

21. Verfahren zur Herstellung einer Verbindung der Formel I

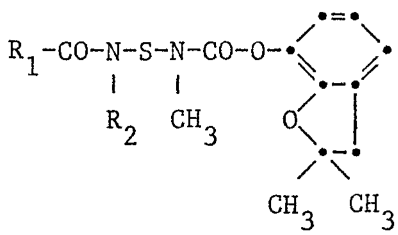

(I),

worin $R_1$ und $R_2$ geradkettiges oder verzweigtes $C_1$-$C_4$-Alkyl oder $C_3$-$C_4$-Cycloalkyl bedeuten, dadurch gekennzeichnet, dass man in Gegenwart einer Base eine Verbindung der Formel II

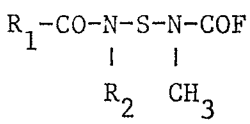

(II)

mit der Verbindung der Formel III

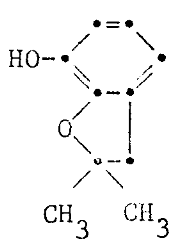

(III)

umsetzt, wobei in Formel II die Reste $R_1$ und $R_2$ die oben angegebenen Bedeutungen haben.

22. Verfahren nach Anspruch 21 zur Herstellung einer Verbindung der Formel Ia gemäss Anspruch 14 bis 20, wobei $R_1$ die in Anspruch 14 bis 16 angegebenen Bedeutungen hat.

23. Verbindung der Formel II

$$R_1 - CO - \underset{\displaystyle R_2}{\underset{|}{N}} - S - \underset{\displaystyle CH_3}{\underset{|}{N}} -CO-F \qquad (II)$$

worin $R_1$ und $R_2$ geradkettiges oder verzweigtes $C_1$-$C_4$-Alkyl oder $C_3$-$C_4$-Cycloalkyl bedeuten.

0090776

Europäisches Patentamt

# EUROPÄISCHER RECHERCHENBERICHT

Nummer der Anmeldung

EP 83 81 0124

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| D,X Y | US-H- 977 008 (C.P. DI SANZO) * Insgesamt * | 1-23 | C 07 D 307/86<br>A 01 N 47/24<br>C 07 C 161/00 |
| X,Y | GB-A-1 512 910 (UNION CARBIDE) * Insgesamt * | 1-23 | |
| D,Y | US-A-3 812 174 (M.S. BROWN) * Insgesamt * | 1-23 | |

RECHERCHIERTE SACHGEBIETE (Int. Cl. 3)

C 07 D 307/00
A 01 N 47/00
C 07 C 161/00

Der vorliegende Recherchenbericht wurde für alle Patentansprüche erstellt.

| Recherchenort | Abschlußdatum der Recherche | Prüfer |
|---|---|---|
| DEN HAAG | 20-06-1983 | ALLARD M.S. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03.82